(19) Europäisches Patentamt
European Patent Office
Office européen des brevets

(11) **EP 3 670 446 A1**

(12) **EUROPEAN PATENT APPLICATION**
published in accordance with Art. 153(4) EPC

(43) Date of publication:
**24.06.2020 Bulletin 2020/26**

(21) Application number: **18845969.7**

(22) Date of filing: **02.08.2018**

(51) Int Cl.:
**C01B 25/32** (2006.01)  **A61L 27/12** (2006.01)
**A61L 27/40** (2006.01)  **A61L 27/56** (2006.01)
**C04B 35/00** (2006.01)  **C04B 38/10** (2006.01)
**C04B 41/85** (2006.01)

(86) International application number:
**PCT/JP2018/028960**

(87) International publication number:
**WO 2019/035361 (21.02.2019 Gazette 2019/08)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **17.08.2017 JP 2017157578**

(71) Applicant: **Shiraishi Central Laboratories Co. Ltd. Amagasaki-shi, Hyogo 660-0085 (JP)**

(72) Inventors:
• **UMEMOTO, Shota**
  **Amagasaki-shi**
  **Hyogo 660-0085 (JP)**
• **TAJIKA, Masahiko**
  **Amagasaki-shi**
  **Hyogo 660-0085 (JP)**
• **UNUMA, Hidero**
  **Yonezawa-shi**
  **Yamagata 992-8510 (JP)**

(74) Representative: **Ter Meer Steinmeister & Partner Patentanwälte mbB**
  **Nymphenburger Straße 4**
  **80335 München (DE)**

(54) **APATITE BODY AND PREPARING METHOD THEREOF**

(57) Provided are an apatite body easily producible and having a stable apatite composition and a method for producing the apatite body. The apatite body is formed of a sintered calcium carbonate body transformed at least at a surface into apatite and the sintered calcium carbonate body may be a porous sintered body.

[FIG. 3]

**Description**

Technical Field

The present invention relates to apatite bodies transformed at least at their surfaces into apatite and methods for producing the same.

Background Art

Apatite is very useful as a bioaffinity artificial biomaterial suitable for bone substitutes, artificial bones, and the like. When apatite is made porous in the case of implantation of the apatite into a living body, the body tissues easily infiltrate a biological implant material. Therefore, there is a demand for production of a porous sintered apatite body.
Patent Literature 1 discloses a method for producing a porous sintered hydroxyapatite body using calcium phosphate-based powder. In an example in Patent Literature 1, a porous sintered hydroxyapatite body is produced using hydroxyapatite powder.

Citation List

Patent Literature

Patent Literature 1: JP-A-2004-115297

Summary of Invention

Technical Problem

However, in producing a sintered apatite body using apatite powder, the production process is complicated and the composition of apatite may change during firing and so on, which presents, for example, a problem that a sintered apatite body having a desired composition may not be able to be obtained.
An object of the present invention is to provide an apatite body easily producible and having a stable apatite composition and a method for producing the apatite body.

Solution to Problem

An apatite body according to the present invention is formed of a sintered calcium carbonate body transformed at least at a surface into apatite.
In the present invention, the sintered calcium carbonate body may be a porous sintered body.
A production method according to the present invention is a method for producing an apatite body, the method including the steps of: making a sintered calcium carbonate body; and reacting the sintered calcium carbonate body with a solution of a phosphate or a solution of a phosphoric acid to transform at least a surface of the sintered calcium carbonate body into apatite.
In the production method according to the present invention, the step of making a sintered calcium carbonate body may include the steps of: making a compacted green body of calcium carbonate; and sintering the compacted green body to produce the sintered calcium carbonate body. In this case, the compacted green body may be a compacted green body of a mixture of calcium carbonate and a sintering aid. Alternatively, the compacted green body may be a compacted green body of calcium carbonate having a purity of 99.7% by mass or more.
In the production method according to the present invention, when the sintered calcium carbonate body is a porous sintered body, the step of making a sintered calcium carbonate body may include the steps of: preparing a dispersion liquid containing calcium carbonate and a gelling agent; adding a foaming agent into the dispersion liquid and then stirring the dispersion liquid until foamy to make a foam; gelling the foam; and sintering the gelled foam to produce a porous sintered body.
In the production method according to the present invention, when the sintered calcium carbonate body is a porous sintered body, the step of making a sintered calcium carbonate body may include the steps of: preparing a dispersion liquid containing calcium carbonate; adding a foaming agent into the dispersion liquid and then stirring the dispersion liquid until foamy to make a foam; and sintering the foam to produce a porous sintered body. In this case, the foam may be freeze-dried and then sintered.

Advantageous Effects of Invention

The present invention enables provision of an apatite body easily producible and having a stable apatite composition and a method for producing the apatite body.

Brief Description of Drawings

[Fig. 1] Fig. 1 is a graph showing respective X-ray diffraction charts of apatite bodies in examples of the present invention.
[Fig. 2] Fig. 2 is a scanning electron micrograph (at 100-fold magnification) showing the surface of the apatite body one day after being immersed into a phosphate aqueous solution.
[Fig. 3] Fig. 3 is a scanning electron micrograph (at 5000-fold magnification) showing the surface of the apatite body one day after being immersed into the phosphate aqueous solution.
[Fig. 4] Fig. 4 is a scanning electron micrograph (at 12000-fold magnification) showing the surface of the apatite body one day after being immersed into the phosphate aqueous solution.
[Fig. 5] Fig. 5 is a scanning electron micrograph (at 100-fold magnification) showing the surface of an apatite body fourteen days after being immersed into a phosphate aqueous solution.
[Fig. 6] Fig. 6 is a scanning electron micrograph (at 5000-fold magnification) showing the surface of the apatite body fourteen days after being immersed into the phosphate aqueous solution.
[Fig. 7] Fig. 7 is a scanning electron micrograph (at 12000-fold magnification) showing the surface of the apatite body fourteen days after being immersed into the phosphate aqueous solution. Description of Embodiments

Hereinafter, a description will be given of a preferred embodiment. However, the following embodiment is merely illustrative and the present invention is not limited to the following embodiment.

<Calcium Carbonate>

No particular limitation is placed on the type of calcium carbonate for use in the present invention so long as it can be used for production of a sintered calcium carbonate body. From the viewpoint of enabling the making and sintering of a high-density green body, the preferred calcium carbonate is one having an average particle diameter ($D_{50}$) in a range of 0.05 to 0.30 $\mu$m in a particle diameter distribution measured by transmission electron microscope observation, a 90% particle diameter ($D_{90}$) of 3 $\mu$m or less in a particle diameter distribution measured by the laser diffraction particle size distribution measurement method, and a BET specific surface area of 5 to 25 $m^2$/g.
The average particle diameter ($D_{50}$) in the particle diameter distribution measured by transmission electron microscope observation is preferably in a range of 0.05 to 0.30 $\mu$m, more preferably in a range of 0.08 to 0.25 $\mu$m, and still more preferably in a range of 0.10 to 0.20 $\mu$m. When the average particle diameter ($D_{50}$) is in the above range, a high-density green body can be made, so that a high-density sintered calcium carbonate body can be produced. The particle diameter distribution by transmission electron microscope observation can be obtained by measuring 1000 or more particles of calcium carbonate, which is an object to be measured, by transmission electron microscope observation.
The 90% particle diameter ($D_{90}$) in the particle diameter distribution measured by the laser diffraction particle size distribution measurement method is preferably 3 $\mu$m or less, more preferably 2.5 $\mu$m or less, and still more preferably 2.0 $\mu$m or less. By determining a particle diameter distribution by the laser diffraction particle size distribution measurement method, the particle diameter distribution of agglomerates of calcium carbonate can be determined. Calcium carbonate having an average particle diameter ($D_{50}$) in the above range in a particle diameter distribution measured by transmission electron microscope observation and having a 90% particle diameter ($D_{90}$) in the above range in a particle diameter distribution measured by the laser diffraction particle size distribution measurement method has a sharp particle diameter distribution and excellent powder packability during forming. Therefore, a high-density green body can be made, so that a high-density sintered calcium carbonate body can be produced.
Furthermore, in the present invention, the ratio ($D_{90}/D_{10}$) of 90% particle diameter ($D_{90}$) to 10% particle diameter ($D_{10}$) in the particle diameter distribution measured by transmission electron microscope observation is preferably 2.3 or less, more preferably 2.2 or less, and still more preferably 2.1 or less. When $D_{90}/D_{10}$ is in the above range, the particle diameter distribution is sharper and the densities of the green body and the sintered calcium carbonate body can be further increased.
Calcium carbonate for use in the present invention can be produced, for example, by a commonly well-known carbon dioxide synthesis method of blowing carbon dioxide into lime milk to react them with each other. In particular, particles having an average particle diameter ($D_{50}$) of over 0.1 $\mu$m can be produced according to the production method described in Japanese Patent No. 0995926.

The BET specific surface area of calcium carbonate for use in the present invention is preferably 5 to 25 m$^2$/g, more preferably 7 to 20 m$^2$/g, and still more preferably 8 to 15 m$^2$/g. When the BET specific surface area is in the above range, the sinterability of calcium carbonate can be increased. Thus, a high-density sintered calcium carbonate body can be produced.

The purity of calcium carbonate for use in the present invention is preferably 99.0% by mass or more, more preferably 99.5% by mass or more, and still more preferably 99.6% by mass or more.

In the present invention, high-purity calcium carbonate having a purity of 99.7% by mass or more can be used. With the use of such high-purity calcium carbonate, the amount of sintering aid necessary for sintering can be made small. Alternatively, sintering can be performed without using any sintering aid. In this relation, high-purity calcium carbonate is preferably one having a purity of 99.8% by mass or more, more preferably one having a purity of 99.9% by mass or more, and still more preferably one having a purity of 99.95% by mass or more. Such high-purity calcium carbonate can be produced, for example, by the method disclosed in Japanese Patent Application Gazette No. 2012-240872.

Although no particular limitation is placed on the upper limit of the purity of high-purity calcium carbonate, it is generally 99.9999% by mass.

<Sintered Calcium Carbonate body>

Examples of the sintered calcium carbonate body for use in the present invention include a porous sintered body and a bulk sintered body. With the use of a porous sintered body, a porous apatite body can be produced.

Examples of the method for producing a porous sintered body include first and second production methods to be described below.

<First Production Method of Porous Sintered body>

The first production method includes the steps of: preparing a dispersion liquid containing calcium carbonate and a gelling agent; adding a foaming agent into the dispersion liquid and then stirring the dispersion liquid until foamy to make a foam; gelling the foam; and sintering the gelled foam to produce a porous sintered body. A detailed description of the first production method will be given below.

(Preparation of Dispersion Liquid)

The dispersion liquid contains calcium carbonate and a gelling agent. When the dispersion liquid contains a gelling agent, the strength of bubbles in a dispersion foam obtained after foaming can be increased to stabilize the shape of the foam. Examples of the gelling agent include polysaccharides, such as methylcellulose, and alkaline hydrosoluble polymers, such as isobutylene/maleic anhydride copolymer.

The content of gelling agent in the dispersion liquid is preferably in a range of 0.1 to 5 parts by mass and more preferably in a range of 0.5 to 3 parts by mass, relative to 100 parts by mass of calcium carbonate. If the content of gelling agent is too small, the strength of bubbles in the foam does not increase, so that the shape of the foam may not be able to be stabilized. If the content of gelling agent is too large, the above effect proportional to the content thereof may not be able to be achieved.

The dispersion liquid is preferably prepared by dispersing calcium carbonate into a dispersion medium, such as water, using a device having a high stirring force, such as a disperser, a mixer or a ball mill, while gradually adding calcium carbonate into the dispersion medium. When a sintering aid is necessary, it is generally added into the dispersion liquid. The content of calcium carbonate is generally preferably 30 to 70% by mass in the dispersion liquid. In doing so, if necessary, about 0 to about 3 parts by mass of polymeric surfactant, such as a polyacrylate, may be added as a dispersant to 100 parts by mass of calcium carbonate.

The gelling agent can be added into the dispersion medium before, after or concurrently with the addition of calcium carbonate.

(Sintering Aid)

Any sintering aid can be used without any particular limitation so long as it enables sintering of calcium carbonate to produce a sintered body. Examples of the sintering aid include those containing carbonates of at least two of lithium, sodium, and potassium and having a melting point of 600°C or lower. The melting point of the sintering aid is preferably 550°C or lower, more preferably 530°C or lower, and still more preferably in a range of 450 to 520°C. When the melting point of the sintering aid is in the above range, calcium carbonate can be fired at a lower temperature to produce a sintered calcium carbonate body. Because in the sintering the sintering aid is used by addition to calcium carbonate, the actual melting point becomes lower than the above temperature and, therefore, it sufficiently acts as a sintering aid. The sintering aid is preferably a mixture of potassium carbonate and lithium carbonate. For example, the melting point of the sintering aid can be determined from a phase diagram or can be measured by differential thermal analysis (DTA).

Alternatively, other examples of the sintering aid include those containing fluorides of at least two of lithium, sodium, and potassium and having a melting point of 600°C or lower. These sintering aids also preferably have the above range of melting points. Examples of these sintering aids include mixtures of potassium fluoride, lithium fluoride, and sodium fluoride. Specifically, examples include mixtures having a composition range of 10 to 60% by mole potassium fluoride, 30 to 60% by mole lithium fluoride, and 0 to 30% by mole sodium fluoride. Within the above range, calcium carbonate can be fired at a lower temperature and a porous sintered calcium carbonate body with a dense wall can be produced.

The content of the sintering aid is, relative to the total amount of calcium carbonate and the sintering aid, preferably in a range of 0.1 to 3.0% by mass, more preferably in a range of 0.2 to 2.5% by mass, and still more preferably in a range of 0.3 to 2.0% by mass. If the content of the sintering aid is too small, calcium carbonate may not sufficiently be sintered. If the content of the sintering aid is too large, the density of the wall of the porous sintered calcium carbonate body may not be able to be increased.

With the use of high-purity calcium carbonate as described previously, the amount of sintering aid necessary for sintering can be made small. Alternatively, sintering can be performed without using any sintering aid.

(Making of Foam)

A foaming agent is added to the above dispersion liquid and the mixture is then stirred until foamy, thus making a foam. Examples of the foaming agent include alkyl sulfate ester salts, such as triethanolamine lauryl sulfate, polyoxyethylene alkyl ether sulfate ester salts, polyoxyethylene alkyl ether acetates, and alkyl polyglucoside.

The addition of the foaming agent is performed so that the concentration of the foaming agent in the dispersion liquid preferably reaches about 0.01 to about 5% by mass and more preferably reaches about 0.1 to about 3% by mass. The stirring is preferably performed with a handheld mixer, a disperser or the like. When the stirring is performed, the temperature of the dispersion liquid may increase. If necessary, the stirring may be performed with cooling of the dispersion liquid.

(Gelation of Foam)

The foam made in the above manner is turned into a gel. By turning the foam into a gel, the shape of the foam can be retained during sintering. Examples of the method for turning the foam into a gel include a method of turning the foam into a gel by making a cross-linked structure with calcium ions in the dispersion liquid and a method of accelerating gelation using temperature properties of the gelling agent itself.

The gelled foam is preferably dried to remove at least some moisture in the foam and then sintered. The drying temperature is preferably in a range of 30 to 200°C.

(Sintering of Foam)

The gelled form is sintered to produce a porous sintered calcium carbonate body. In the present invention, the foam is preferably presintered and then finally sintered. Thus, it can be prevented that organic components contained in the foam remain and become carbonized and darkened or the organic components rapidly decompose to create cracks in the sintered body.

The temperature of the presintering is preferably in a range of 200 to 500°C and more preferably in a range of 300 to 420°C. The temperature of the final sintering is preferably equal to or higher than the temperature of the presintering and in a range of 420 to 600°C, and more preferably in a range of 450 to 540°C.

Furthermore, the rate of temperature increase during the presintering and the final sintering is preferably in a range of 2°C to 20°C per minute. Thus, it can be prevented that the organic components rapidly decompose to create cracks in the sintered body.

The atmosphere during the sintering is preferably in air. However, the present invention is not limited to this and the foam may be sintered in a carbon dioxide atmosphere or in an atmosphere of inert gas, such as nitrogen gas.

<Second Production Method of Porous Sintered body>

The second production method includes the steps of: preparing a dispersion liquid containing calcium carbonate; adding a foaming agent into the dispersion liquid and then stirring the dispersion liquid until foamy to make a foam; and sintering the foam to produce a porous sintered body. A detailed description of the second production method will be given below.

(Preparation of Dispersion Liquid)

Calcium carbonate is preferably dispersed into a dispersion medium, such as water, using a device having a high stirring force, such as a disperser, a mixer or a ball mill, with gradual addition of calcium carbonate into the dispersion medium. The content of calcium carbonate is generally preferably 30 to 70% by mass in the dispersion liquid. In doing so, if necessary, about 0 to about 3 parts by mass of polymeric surfactant, such as a polyacrylate, may be added as a dispersant to 100 parts by mass of calcium carbonate.

When a sintering aid is necessary, it is generally added into the dispersion liquid. The sintering aids that can be used are the same as in the first production method. The sintering aid can be added into the dispersion liquid in the same manner as in the first production method.

(Excipient)

An excipient may be added into the above dispersion liquid. The addition of an excipient can increase the strength of bubbles in a dispersion foam obtained after foaming to stabilize the shape of the foam. Examples of the excipient include starch, dextrin, polyvinyl alcohol, polypropylene glycol, pectin, alginic acids, and sodium salts of carboxycellulose.

(Making of Foam)

A foaming agent is added to the above dispersion liquid and the mixture is then stirred until foamy, thus making a foam. The addition of the foaming agent is preferably performed so that the concentration of the foaming agent in the dispersion liquid reaches about 0.01 to about 5% by mass. The stirring is preferably performed with a handheld mixer, a disperser or the like. When the stirring is performed, the temperature of the dispersion liquid may increase. If necessary, the stirring may be performed with cooling of the dispersion liquid. The foaming agents that can be used are the same as in the first production method.

(Freeze-Drying)

The above foam is preferably freeze-dried and then sintered. By the freeze-drying, the shape of the foam can be easily retained, so that a porous sintered body can be obtained in a good shape.

Specifically, it is preferred that the foam should be preliminarily frozen at -40°C or lower under ordinary pressure for two hours or more and then gradually increased in temperature under reduced pressure while its ice crystals are sublimated. The condition of the reduced pressure is preferably at 20 Pa or less and more preferably at 10 Pa or less. The temperature is preferably gradually increased while the reduced pressure is maintained without melting the ice crystals, and the temperature is generally controlled in a range of -40°C to 60°C.

(Sintering of Foam)

When the foam obtained in the above manner is sintered in the same manner as in the first production method, a porous sintered body can be produced.

<Porous Sintered Calcium Carbonate Body>

The porosity of the porous sintered calcium carbonate body is preferably 50% by volume or more, more preferably 60% by volume or more, still more preferably 70% by volume or more, yet still more preferably 80% by volume or more, and particularly preferably 85% by volume or more. Thus, the porous sintered calcium carbonate body becomes available also for biological and like applications. Although no particular limitation is placed on the upper limit of the porosity of the porous sintered calcium carbonate body, it is generally 95% by volume.

In the porous sintered calcium carbonate body, a connected pore leading to the exterior of the sintered body is preferably formed. In a porous apatite body obtained by transforming the surface of the porous sintered calcium carbonate body into apatite, apatite in the internal surface can be easily brought into contact with the outside. Thus, apatite in the surface can have its effect more efficiently.

The purity of the porous sintered calcium carbonate body obtained using high-purity calcium carbonate is preferably 99.7% by mass or more, more preferably 99.8% by mass or more, still more preferably 99.9% by mass or more, yet still more preferably 99.95% by mass or more, and particularly preferably 99.99% by mass or more . Thus, the porous sintered calcium carbonate body becomes available also for biological and like applications. Although no particular limitation is placed on the upper limit of the purity of the porous sintered calcium carbonate body, it is generally 99.9999% by mass.

<Method for Producing Bulk Sintered Body>

Amethod for producing a bulk sintered body includes the steps of: making a compacted green body of calcium carbonate; and sintering the compacted green body to produce a sintered calcium carbonate body. A detailed description of the method for producing a bulk sintered body will be given below.

(Making of Compacted Green Body)

A mixture of calcium carbonate powder and a sintering aid or calcium carbonate powder only is compacted to make a green body. The compaction is preferably uniaxial pressing. With the use of calcium carbonate having an average particle diameter ($D_{50}$) in a range of 0.05 to 0.30 $\mu$m, a high-purity sintered calcium carbonate body having a high density can be produced even using a green body made by uniaxial pressing. However, in the present invention, the making of a green body is not limited to uniaxial pressing and a green body may be made by any other known forming method, such as isostatic pressing, doctor blade technique or casting.

The sintering aids that can be used are the same as in the first and second production methods. The sintering aid can be used in the same proportion as in the first and second production methods . With the use of high-purity calcium carbonate, the amount of sintering aid necessary for sintering can be made small. Alternatively, sintering can be performed without using any sintering aid.

The relative density of the green body is preferably 50% or more, more preferably 55% or more, and still more preferably 58% or more. The relative density of the green body is a value obtained by dividing the bulk density of the green body by the theoretical density (2.711 g/cm$^3$) of calcium carbonate. The bulk density of the green body can be measured by the Archimedes' method to be described later. The relative density of the green body is preferably that obtained when the mixture is uniaxially pressed at a forming pressure of 196.1 Mpa (2000 kgf/cm$^2$). Within the above range of relative densities, a higher-density sintered calcium carbonate body can be obtained.

(Sintering of Compacted Green Body)

The above green body is sintered to produce a bulk sintered body. From the viewpoint of sintering in a simpler process, the atmosphere during the sintering is preferably in air. However, the present invention is not limited to this and the green body may be sintered in a carbon dioxide atmosphere or in an atmosphere of inert gas, such as nitrogen gas. With the use of calcium carbonate having an average particle diameter ($D_{50}$) in a range of 0.05 to 0.30 $\mu$m, a bulk sintered body having a high density can be produced even by sintering in air.

The sintering temperature is preferably 600°C or lower, more preferably 580°C or lower, and still more preferably 560°C or lower. If the sintering temperature is too high, calcium carbonate is likely to decompose to generate calcium oxide, which is undesirable. The sintering temperature is preferably not lower than 420°C, more preferably not lower than 430°C, and still more preferably not lower than 440°C. If the sintering temperature is too low, calcium carbonate may not sufficiently be sintered.

The relative density of the bulk sintered body is preferably 90% or more, more preferably 95% or more, still more preferably 97% or more, yet still more preferably 98% or more, and particularly preferably 99% or more.

The purity of the bulk sintered body obtained by using high-purity calcium carbonate is preferably 99.7% by mass or more, more preferably 99.8% by mass or more, still more preferably 99.9% by mass or more, yet still more preferably 99.95% by mass or more, and particularly preferably 99.99% by mass or more. Although no particular limitation is placed on the upper limit of the purity, it is generally 99.9999% by mass.

<Apatite Body>

An apatite body according to the present invention can be produced by reacting the sintered calcium carbonate body obtained in the above manner with a solution of a phosphate or a solution of a phosphoric acid to transform at least the surface of the sintered calcium carbonate body into apatite.

Examples of the phosphate include triammonium phosphate, tripotassium phosphate, trisodium phosphate, ammonium disodium phosphate, diammonium sodium phosphate, ammonium dihydrogen phosphate, potassium dihydrogen phosphate, sodium dihydrogen phosphate, trimagnesium phosphate, sodium ammonium hydrogen phosphate, diammonium hydrogen phosphate, dipotassium hydrogen phosphate, disodium hydrogen phosphate, magnesium hydrogen phosphate-tridiacetyl phosphate, diphenyl phosphate, dimethyl phosphate, cellulose phosphate, ferrous phosphate, ferric phosphate, tetrabutylammonium phosphate, copper phosphate, triethyl phosphate, tricresyl phosphate, tris(trimethylsilyl) phosphate, triphenyl phosphate, tributyl phosphate, trimethyl phosphate, guanidine phosphate, and cobalt phosphate. These types of phosphates may be used in various combinations of two or more. Preferred phosphates among them are primary to tertiary phosphates $M_{3-x}H_xPO_4$ (where M is Na, K or NH4 and x is an integer from 0 to 2). The primary to tertiary phosphates may be used in a mixture for the purpose of adjusting the pH.

Examples of the phosphoric acid include orthophosphoric acid, pyrophosphoric acid, and condensed phosphoric acid and orthophosphoric acid is preferably used.

A solution for use with the phosphate or the phosphoric acid is generally an aqueous solution. The concentration of the phosphate or the phosphoric acid is preferably in a range of 0.001 to 3 mol/L, more preferably in a range of 0.01 to 2 mol/L, and still more preferably in a range of 0.1 to 1 mol/L. The temperature of the solution of phosphate or phosphoric acid is preferably in a range of 10 to 100°C and more preferably in a range of 60 to 90°C. Furthermore, in the case where a pressure-resistant container or the like is used, the sintered calcium carbonate body can be immersed into the solution even in a range of 100 to 280°C.

Generally, the sintered calcium carbonate body is immersed into the solution of phosphate or phosphoric acid to react the sintered calcium carbonate body with the phosphate or phosphoric acid and thus transform the surface of the sintered calcium carbonate body into apatite. However, the method for reacting the sintered calcium carbonate body with the solution of phosphate or phosphoric acid is not limited to this and any method can be used so long as it enables the surface of the sintered calcium carbonate body to be brought into contact with the solution of phosphate or phosphoric acid to react with the phosphate or the phosphoric acid.

No particular limitation is placed on the time for immersing of the sintered calcium carbonate body into the solution of phosphate or phosphoric acid and, for example, the sintered calcium carbonate body can be immersed into the solution in a range of an hour to four weeks and preferably in a range of three days to two weeks.

After the immersion, the apatite body is picked up and, as necessary, subjected to washing with water, drying, and so on. Examples

Hereinafter, a description will be given of specific examples according to the present invention, but the present invention is not limited to the following examples.

<Example 1>

(Production of Porous Sintered Calcium Carbonate Body: Second Production Method)

As calcium carbonate, calcium carbonate having a purity of 99.99% by mass, an average particle diameter ($D_{50}$) of 0.15 $\mu$m, and a BET specific surface area of 10 m$^2$/g was used. The purity was derived by the difference method. Specifically, the respective amounts of impurities in a sample liquid for measurement in which a sample of known mass was dissolved were measured with an inductively coupled plasma emission spectrometer, the sum of the measurement results was considered as the content of impurities, and a value obtained by subtracting the content of impurities from the total mass was defined as the purity. The average particle diameter ($D_{50}$) was determined by measuring the particle diameters of 1500 particles of calcium carbonate, which is an object to be measured, by transmission electron microscope observation and using the obtained particle diameter distribution. The BET specific surface area was measured by the single point method using FlowSorb 2200 manufactured by Shimadzu Corporation.

Pure water was put into a polyethylene bottle containing a suitable amount of zirconia balls and the above-described calcium carbonate was added into the pure water to reach 39% by volume. Next, 0.8 parts by mass of polyvinyl alcohol as an excipient and 2.5 parts by mass of polymeric surfactant as a dispersant (special polycarboxylate polymer type surfactant under the trade name of "POIZ 520" manufactured by Kao Corporation) were added to 100 parts by mass of calcium carbonate and the mixture was then wet mixed for 12 hours using a pot mill. A 19% by mass aqueous solution of polyoxyethylene alkyl ether as a foaming agent was added to the obtained slurry to reach 2 ml per 10 g of slurry, thus preparing a dispersion liquid.

The dispersion liquid was foamed with a handheld mixer to obtain a foam. The obtained foam was poured into a mold and freeze-dried in this state. The freeze-drying was performed under the conditions that the foam was preliminarily frozen at minus 40°C under ordinary pressure for 12 hours and then held at 30°C under a reduced pressure of 10 Pa for 48 hours.

The freeze-dried foam was increased in temperature at a rate of 10°C per minute until a presintering temperature (350°C) and presintered for 10 hours after the temperature increase. After having been cooled, the foam was increased in temperature at the same rate of temperature increase until a final sintering temperature (510°C) and finally sintered for three hours after the temperature increase, thus obtaining a porous sintered calcium carbonate body.

The purity of the obtained porous sintered calcium carbonate body was 99.9% by mass and the porosity thereof was 89.0%. The purity was measured by the above-described difference method. The porosity was obtained by cutting the sintered body into a rectangular block, determining the density of the block from the weight and apparent volume of the block, dividing the density by the true density of calcium carbonate, 2.711 g/cm$^3$, to obtain a relative density, and defining as the porosity a value obtained by subtracting the relative density from the entirety.

(Production of Apatite Body)

The obtained porous sintered calcium carbonate body was immersed into an aqueous solution of disodium hydrogen phosphate ($Na_2HPO_4$) having a concentration of 1 mol/L and held at 60°C, thus transforming the surface of the sintered calcium carbonate body into apatite to produce an apatite body. By using different immersion times including one day, three days, five days, seven days, and fourteen days, apatite bodies different in immersion time were produced.

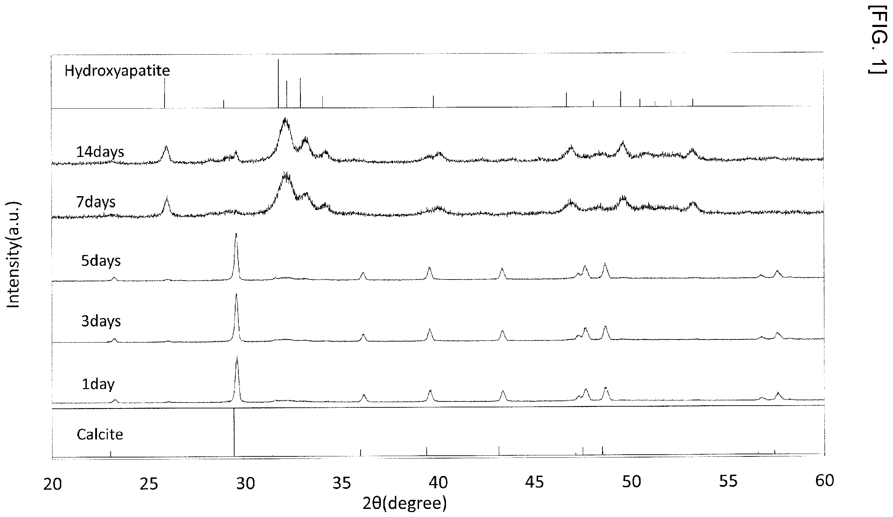

Fig. 1 is a graph showing respective X-ray diffraction charts of the apatite bodies. Fig. 1 also shows the positions of X-ray diffraction peaks of hydroxyapatite and calcium carbonate (calcite).

As shown in Fig. 1, the apatite body obtained by immersion for seven days and the apatite body obtained by immersion for fourteen days exhibited lowered calcium carbonate peaks and raised hydroxyapatite peaks, which shows that a major portion of the porous sintered calcium carbonate body was transformed into apatite.

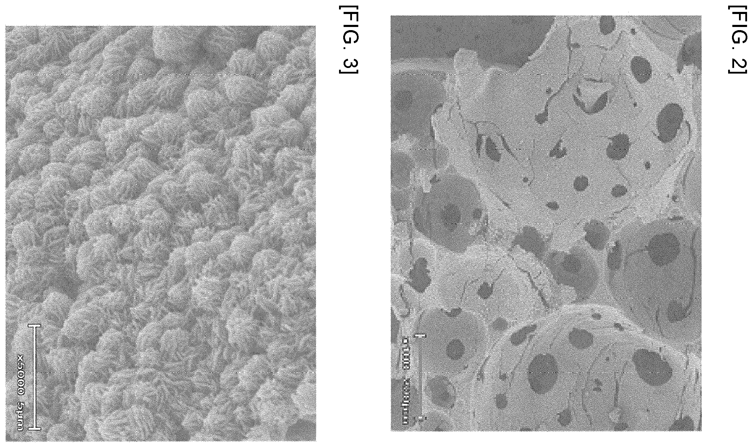

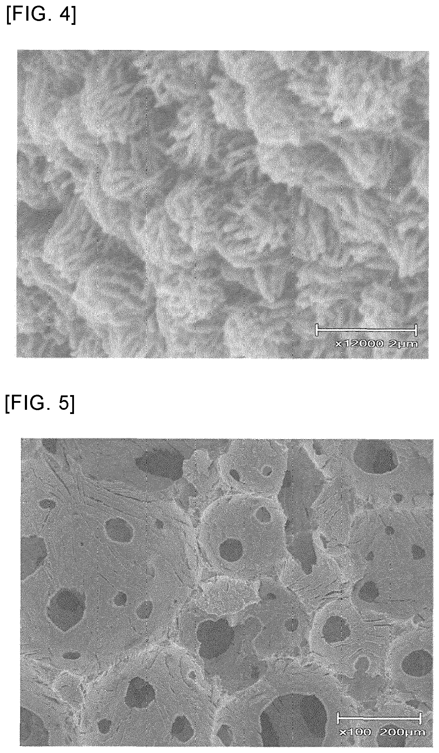

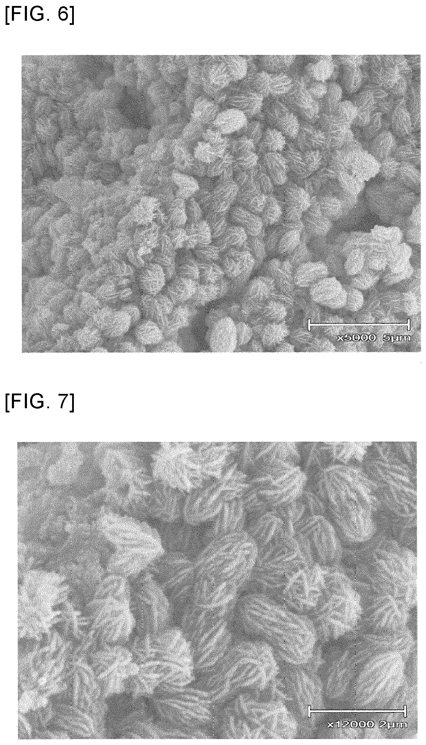

Fig. 2 (at 100-fold magnification), Fig. 3 (at 5000-fold magnification), and Fig. 4 (at 12000-fold magnification) are scanning electron micrographs showing the apatite body obtained by immersion for one day. Fig. 5 (at 100-fold magnification), Fig. 6 (at 5000-fold magnification), and Fig. 7 (at 12000-fold magnification) are scanning electron micrographs showing the apatite body obtained by immersion for fourteen days.

As shown in Figs. 3 and 4, whisker-like projections were observed on the surface of the apatite body obtained by immersion for one day. Such whisker-like projections were not observed on the porous sintered calcium carbonate body not transformed at the surface into apatite. Therefore, it can be considered that the whisker-like projections were produced by transforming the surface of the porous sintered calcium carbonate body into apatite. Hence, it was confirmed that the surface of the apatite body obtained by immersion for one day was transformed into apatite.

(Protein Adsorption Experiment)

As samples, the above-described porous sintered calcium carbonate body and an apatite body obtained by immersing the porous sintered calcium carbonate body for 67 hours into an aqueous solution of disodium hydrogen phosphate of the same type as described above were used. These samples were subjected to a protein adsorption experiment in the following manner. The samples were used in a form ground in a mortar.

    (1) An amount of 10 mg of sample was weighed off into a polypropylene tube.
    (2) A 1 ml solution of lysozyme at a concentration of 1 mg/ml was added into the tube.
    (3) The tube was stirred with a touch mixer and then shaken with a shaker for an hour.
    (4) The shaken tube was centrifuged to settle out the sample and 80 microliters of supernatant liquid was weighed out into another tube.
    (5) A 4 ml solution for protein determination (a protein assay manufactured by Bio-Rad Laboratories) was added into the tube, followed by mixing with a touch mixer and then standing still.
    (6) After the standing, the tube was put into a spectrophotometric cell and the supernatant liquid was measured in terms of light absorbance at a wavelength of 595 nm. In doing so, protein contained in the supernatant liquid of the sample reacts with the solution for protein determination to cause a color reaction, so that the light absorbance in a wavelength range around 595 nm increases.

While the light absorbance in the case of the porous sintered calcium carbonate body was 0.5984%, the light absorbance in the case of the apatite body was 0.5319% smaller than that in the case of the porous sintered calcium carbonate body. From these results, it was confirmed that an apatite body transformed at the surface into apatite has a larger amount of protein adsorbed than a porous sintered calcium carbonate body not transformed at the surface into apatite.

When these samples were measured in terms of BET specific surface area, the BET specific surface area of the porous sintered calcium carbonate body was 0.7 $m^2/g$ and the BET specific surface area of the apatite body was 3.5 $m^2/g$.

<Example 2>

(Production of Porous Sintered Calcium Carbonate Body: First Production Method)

As calcium carbonate, calcium carbonate having a purity of 99.61% by mass, an average particle diameter ($D_{50}$) of 0.15 $\mu$m, and a BET specific surface area of 10 $m^2/g$ was used.

An amount of 55 parts by mass of ion-exchange water, 100 parts by mass of calcium carbonate described above, 0.55 parts by mass of methylcellulose, 2.5 parts by mass of special polycarboxylate polymer type surfactant (effective number of parts: 1.0 parts by mass), 0.32 parts by mass of potassium carbonate, and 0.28 parts by mass of lithium carbonate were mixed with a homogenizer-disperser, thus obtaining a dispersion liquid. Methylcellulose is a gelling agent, the special carboxylate polymer type surfactant is a dispersant, and potassium carbonate and lithium carbonate are sintering aids.

An amount of 0.97 parts by mass (effective number of parts: 0.39 parts by mass) of triethanolamine lauryl sulfate as a foaming agent was added into the obtained dispersion liquid and the mixture was stirred until foamy at 1000 rpm for 10 minutes with a handheld mixer, thus making a foam.

The foam was put into a forming die made by paper, the forming die was moved into a hot-air dryer, and the foam was heated at 80°C for 0.5 hours in the hot-air dryer to turn the foam into a gel. The gelled foam was heated at 80°C for 12 hours to dry it.

The gelled and dried foam was increased in temperature at a rate of 5°C/min until a presintering temperature (400°C) and presintered for 10 hours after the temperature increase. Next, the foam was increased in temperature at the same rate of temperature increase from 400°C until a final sintering temperature (510°C), finally sintered for three hours after the temperature increase, and then cooled at a rate of 10°C/min until room temperature, thus obtaining a porous sintered calcium carbonate body. The porosity of the obtained porous sintered calcium carbonate body was 82% by volume.

(Production of Apatite Body)

The obtained porous sintered calcium carbonate body was immersed for 67 hours into an aqueous solution of disodium hydrogen phosphate ($Na_2HPO_4$) having a concentration of 1 mol/L and held at 60°C, thus transforming the surface of the calcium carbonate body into apatite to produce an apatite body.

The obtained apatite body was subjected to a protein adsorption experiment in the same manner as described above. Thus, it was confirmed that an apatite body transformed at the surface into apatite has a larger amount of protein adsorbed than a porous sintered calcium carbonate body not transformed at the surface into apatite.

<Example 3>

(Production of Bulk Sintered Calcium Carbonate Body)

As calcium carbonate, calcium carbonate having a purity of 99.99% by mass, an average particle diameter ($D_{50}$) of 0.15 $\mu$m, and a BET specific surface area of 10 $m^2$/g was used. Using this calcium carbonate, a bulk sintered calcium carbonate body was produced in the following manner.

(Making of Green Body)

Calcium carbonate was put into a polyethylene bottle containing a suitable amount of zirconia balls and dry mixed overnight to obtain a raw material powder. This raw material powder was put into a cylindrical die and uniaxially pressed using a press . The raw material powder was preliminarily pressed at a forming pressure of 98 Mpa (1000 kgf/$cm^2$) for one minute and then pressed at a forming pressure of 196.1 Mpa (2000 kgf/$cm^2$) for one minute.

(Firing of Green Body)

The obtained green body was fired at a firing temperature of 540°C in air for three hours to sinter it. Note that until the firing temperature was reached, the temperature was increased at a rate of 10°C per minute. By the firing, a bulk sintered calcium carbonate body was obtained.

(Measurement of Relative Density of Bulk Sintered Calcium Carbonate Body)

The bulk density $\rho_b$[g/$cm^3$] of the sintered calcium carbonate body was obtained by the Archimedes' method and the obtained bulk density was divided by the theoretical density (2.711 g/$cm^3$) of calcium carbonate to obtain its relative density. The bulk density of the sintered calcium carbonate body was obtained as follows. First, the dry weight $W_1$ of a sample of the sintered calcium carbonate body was measured, the sample was allowed to stand for about 10 minutes in paraffin warmed in a vessel put in hot water, then picked up, and cooled to ordinary temperature. After the cooling, the weight $W_2$ of the sample containing paraffin was measured. Thereafter, the weight $W_3$ of the sample in water was measured and the bulk density $\rho_b$ of the sample was then determined from the following equation.

$$\text{Bulk Density } \rho_b \text{ [g/cm}^3\text{]} = W_1\rho_W/(W_2 - W_3)$$

$\rho_W$:  water density [g/cm$^3$]
$W_1$:  dry weight [g] of sample
$W_2$:  weight [g] of sample containing paraffin
$W_3$:  weight [g] of sample in water

The relative density of sintered calcium carbonate body was 97.0%.

(Measurement of Purity of Bulk Sintered Calcium Carbonate Body)

The purity of the sintered calcium carbonate body derived by the above-described difference method was 99.99%.

(Production of Apatite Body)

The obtained bulk sintered calcium carbonate body was immersed for 67 hours into an aqueous solution of disodium hydrogen phosphate ($Na_2HPO_4$) having a concentration of 1 mol/L and held at 60°C, thus transforming the surface of the calcium carbonate body into apatite to produce an apatite body.
The obtained apatite body was subjected to the protein adsorption experiment. Thus, it was confirmed that an apatite body transformed at the surface into apatite has a larger amount of protein adsorbed than a bulk sintered calcium carbonate body not transformed at the surface into apatite.

**Claims**

1.  An apatite body formed of a sintered calcium carbonate body transformed at least at a surface into apatite.

2.  The apatite body according to claim 1, wherein the sintered calcium carbonate body is a porous sintered body.

3.  A method for producing an apatite body, the method comprising the steps of:

    making a sintered calcium carbonate body; and
    reacting the sintered calcium carbonate body with a solution of a phosphate or a solution of a phosphoric acid to transform at least a surface of the sintered calcium carbonate body into apatite.

4.  The method for producing an apatite body according to claim 3, wherein the step of making a sintered calcium carbonate body comprises the steps of:

    making a compacted green body of calcium carbonate; and
    sintering the compacted green body to produce the sintered calcium carbonate body.

5.  The method for producing an apatite body according to claim 4, wherein the compacted green body is a compacted green body of a mixture of calcium carbonate and a sintering aid.

6.  The method for producing an apatite body according to claim 4, wherein the compacted green body is a compacted green body of calcium carbonate having a purity of 99.7% by mass or more.

7.  The method for producing an apatite body according to any one of claims 4 to 6, wherein
    the sintered calcium carbonate body is a porous sintered body, and
    the step of making a sintered calcium carbonate body comprises the steps of:

    preparing a dispersion liquid containing calcium carbonate and a gelling agent;
    adding a foaming agent to the dispersion liquid, followed by stirring until foamy to make a foam;
    turning the foam into a gel; and
    sintering the gelled foam to produce the porous sintered body.

8. The method for producing an apatite body according to any one of claims 4 to 6, wherein
the sintered calcium carbonate body is a porous sintered body, and
the step of making a sintered calcium carbonate body comprises the steps of:

preparing a dispersion liquid containing calcium carbonate;
adding a foaming agent to the dispersion liquid, followed by stirring until foamy to make a foam; and
sintering the foam to produce the porous sintered body.

9. The method for producing an apatite body according to claim 8, wherein the foam is freeze-dried and then sintered.

[FIG. 1]

[FIG. 2]

[FIG. 3]

[FIG. 4]

[FIG. 5]

[FIG. 6]

[FIG. 7]

## INTERNATIONAL SEARCH REPORT

| | International application No. |
|---|---|
| | PCT/JP2018/028960 |

**A. CLASSIFICATION OF SUBJECT MATTER**
Int.Cl. C01B25/32(2006.01)i, A61L27/12(2006.01)i, A61L27/40(2006.01)i, A61L27/56(2006.01)i, C04B35/00(2006.01)i, C04B38/10(2006.01)i, C04B41/85(2006.01)i

According to International Patent Classification (IPC) or to both national classification and IPC

**B. FIELDS SEARCHED**

Minimum documentation searched (classification system followed by classification symbols)
Int.Cl. C01B25/32, A61L27/12, A61L27/40, A61L27/56, C04B35/00, C04B38/10, C04B41/85

Documentation searched other than minimum documentation to the extent that such documents are included in the fields searched
Published examined utility model applications of Japan 1922-1996
Published unexamined utility model applications of Japan 1971-2018
Registered utility model specifications of Japan 1996-2018
Published registered utility model applications of Japan 1994-2018

Electronic data base consulted during the international search (name of data base and, where practicable, search terms used)
CAplus (STN), JSTPlus (JDreamIII), JST7580 (JDreamIII), JSTChina (JDreamIII)

**C. DOCUMENTS CONSIDERED TO BE RELEVANT**

| Category* | Citation of document, with indication, where appropriate, of the relevant passages | Relevant to claim No. |
|---|---|---|
| X<br>A | WO 2017/038360 A1 (GC CORPORATION) 09 March 2017, paragraphs [0015]-[0082], examples, claims & CN 107922277 A | 1-3<br>7-9 |
| X<br>A | JP 2005-112712 A (NIHON UNIVERSITY) 28 April 2005, claims, paragraphs [0004]-[0012], examples (Family: none) | 1, 2<br>7-9 |

☒ Further documents are listed in the continuation of Box C. ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 13 September 2018 (13.09.2018) | 25 September 2018 (25.09.2018) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

| International application No. |
| --- |
| PCT/JP2018/028960 |

C (Continuation).    DOCUMENTS CONSIDERED TO BE RELEVANT

| Category* | Citation of document, with indication, where appropriate, of the relevant passages | Relevant to claim No. |
| --- | --- | --- |
| A | JP 11-240782 A (SEKIZAWA, Nobuyuki) 07 September 1999, paragraph [0011] (Family: none) | 1-9 |
| Y A | JP 11-180705 A (MURAKASHI LIME INDUSTRY CO., LTD.) 06 July 1999, claims, paragraphs [0001], [0004]-[0017], examples (Family: none) | 3-6 7-9 |
| Y A | JP 7-242415 A (INAX CORP.) 19 September 1995, claims, paragraphs [0015]-[0020], examples & DE 19507309 A1 & NL 9500371 A | 3-6 7-9 |
| Y A | JP 2015-065970 A (OLYMPUS CORP.) 13 April 2015, claims, paragraph [0018] (Family: none) | 6 7-9 |
| A | CN 104030718 A (GUNGZHOU MEDICAL UNIVERSITY) 10 September 2014, entire text (Family: none) | 1-9 |
| E, A | JP 2018-140890 A (SHIRAISHI CENTRAL LABORATORIES CO., LTD.) 13 September 2018, entire text (Family: none) | 1-9 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004115297 A
- JP 0995926 A
- WO 2012240872 A